# EUROPEAN PATENT APPLICATION

(11) **EP 0 887 063 A1**
(43) Date of publication of application: **30.12.1998**
(21) Application number: 97110604.2
(22) Date of filing: 28.06.1997
(51) Int. Cl.: A61F 13/15, A61F 5/451

(54) **Faecal collector for a female wearer**

(71) Applicant: THE PROCTER & GAMBLE COMPANY, Cincinnati, Ohio 45202 (US)
(72) Inventor: Palumbo, Gianfranco, 61348 Bad Homburg (DE); D'Acchioli, Vincenzo, 65779 Kelkheim am Taunus (DE)
(74) Representative: Canonici, Jean-Jacques

(57) **Abstract**

The present invention relates to a faecal management device designed for a female wearer. The faecal management device comprises a bag having an aperture, and an anatomically-shaped flange, which surrounds the aperture. The flange provides for adhesive attachment to the perianal area of the wearer. In particular, the front portion of the flange comprises a projection, which fits snugly into the folds at the margin of the vulva of the wearer. In another aspect of the present invention, the faecal management device comprising such a flange is used in combination with a disposable diaper.

## Description

### Field of the invention

The present invention relates to a gender specific faecal management device. In particular, the device is directed towards a female wearer.

### Background of the invention

Faecal management devices are known articles of manufacture that are designed to be worn principally by incontinence sufferers and infants. Such faecal management devices are attached to the anal region of the wearer and are intended to entrap and immediately contain faecal material and other bodily discharges. As a consequence, these devices are functionally effective in eliminating the problem of smearing on the skin of the wearer; in lessening epidermal irritation; in preventing contamination of articles such as clothing and bedding; and even in preventing the soiling of the carers themselves. Nevertheless, a problem often encountered during the use of such faecal management devices is that some of the faecal material can flow into the urethra of a female wearer. Typically, the presence of such faecal material can lead to a nasty infection in the urethra. Such a condition is most undesirable, painful and distressing to the bedridden wearer or to the infant.

Many articles exist on the market that allege the entrapment and immediate containment of faecal material in an effective manner. The prior art is rich with such examples. For instance, WO 96/19167 describes an absorbent article adapted to contain faecal material and highly fluidic material that is expelled at high velocity. The absorbent article comprises faecal containment members positioned transversely outward of the absorbent assembly, which function by interrupting the lateral movement of the faecal material. The faecal containment members are formed of resilient and porous materials to maintain and provide sufficient void volume to collect the faecal material, respectively. WO 94/14395 discloses a disposable absorbent article comprising a transverse partition disposed on the body facing surface of the topsheet and extending outwardly therefrom, to be upstanding and extend away from the plane of the disposable absorbent article. The transverse partition divides the absorbent article into a front portion and a rear portion, and presents an abrupt discontinuity between the two portions. Faecal material deposited in the rear portion is obstructed from longitudinally migrating to the front portion by the transverse partition. WO 97/01316 details a diaper with a bag for collecting faecal material and a urine collector for storing urine. The bag is formed by doubling over the diaper along a central imaginary line (perpendicular to the longitudinal sides of the diaper) and by drawing the edges (spaced apart from the central imaginary line) parallel to the imaginary line together. In such a manner, the faecal material is isolated from the skin of the wearer. Japanese application JP 08-117 261 A discloses a diaper having a bag like structure, which is designed for incontinence sufferers. The bag comprises a slit or a hole in a position facing the anus with the slit or hole being surrounded by a surface coated with adhesive. The bag is formed of a water-repellent material. US 3,577,989 details a disposable plastic elimination-trapping bag for incontinence sufferers. The flange is specifically designed and shaped such that it is generally convex at its curved rearward end while being generally concave at its forward end. In particular, for a female patient, the convex portion of the flange is adapted to closely follow the curved body contour and to fit between the cheeks of the buttocks and below and around the anus while the concave forward end portion of the flange is designed to closely follow the curved contour of the body of the patient above the vulva. Between the end portions of the flange are elongated side portions dimensioned to closely follow the curved body contour between the anus, cheeks and upper legs of the sufferer. European application EP 0 245 064 A2 describes a faecal incontinence bag having flexible front and rear walls secured together around their periphery. The front wall has a hole for entry of the matter discharged by the wearer. The hole is surrounded by an adhesive pad of skin-compatible water-resistant material secured to the external surface of the front wall. The pad is generally heart-shaped so that when in position the concave portion of the heart-shaped pad is towards the front of the wearer. For all the prior art cases, the faecal matter is not entirely contained in the bag of the faecal management device. It is known that some of the faecal material seeps forward from the anal region and creeps into the urethra.

Nonetheless, the need exists for a faecal management device that is extremely effective in completely isolating the faecal material from the sensitive organs of the female anatomy. The present invention addresses this need by providing a projection at the front portion of the flange. It has been found that the presence of such a projection is uniquely advantageous and prevents the flow of faecal material into the urethra of the female wearer. Furthermore, the presence of such a projection on the faecal management device causes no discomfort to the wearer, leads to a great reduction in infections and epidermal irritations derived from faecal material and results in a high level of wearer and carer satisfaction in relation to skin healthiness.

In another aspect of the present invention, the faecal management device with this projection can be advantageously used with a disposable diaper. The prior art is silent on such a combination. As described above, none of the prior art documents discloses two separate entities that work synergetically to isolate the faecal material from the sensitive organs of the female anatomy and which furthermore isolate the skin of the wearer from the absorbent material of the diaper.

### Summary of the invention

A faecal management device constructed in accordance with the present invention comprises a bag having an aperture and an anatomically-shaped flange surrounding the aperture for adhesive attachment to the perianal area of the wearer. The anatomically-shaped flange is attached to the bag and comprises an outer periphery, an inner periphery adjacent to the aperture, a longitudinal centreline and a transverse centreline wherein the transverse centreline segments the flange into a front portion and a rear portion.

In particular, the flange comprises a projection in the front portion. The projection is disposed between the outer periphery and the inner periphery of the flange in a direction parallel to the longitudinal direction. Preferably, the projection extends from the outer periphery to the inner periphery and is disposed in a symmetrical manner. The projection has an effective height ranging from 2 millimetres to 20 millimetres, preferably ranging from 3 millimetres to 10 millimetres, more preferably about 5 millimetres. The projection is adapted to fit snugly between the vulva and the anus, i.e., the perineum of the female wearer. In order to ensure even more conformity with the body of the wearer, the anatomically-shaped flange is heart-shaped.

In another aspect of the present invention, the present faecal management device is used in combination with a disposable diaper.

### Brief description of the drawings

It is believed that the invention will be better understood from the foregoing description in conjunction with the accompanying drawings in which:
Figure 1 is a perspective view of a faecal management device according to the teachings of the present invention;
Figure 2 shows a perspective view of the faecal management device in conjunction with a disposable diaper; and
Figure 3 is a partially cut-away perspective view of a disposable diaper embodying the present invention.

### Detailed description of the invention

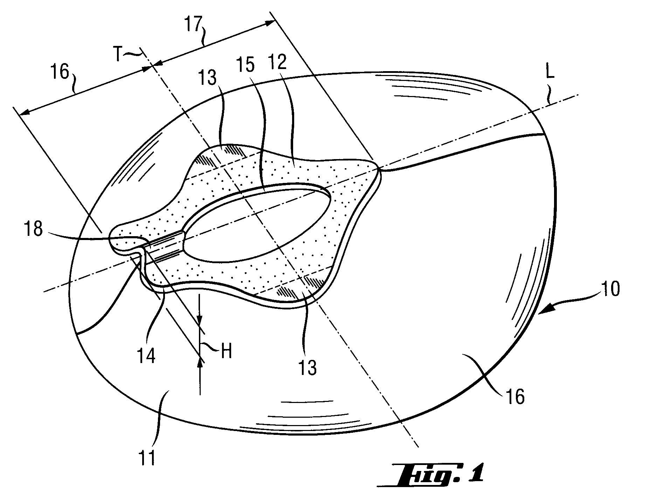

According to Figure 1, the faecal management device **10** of the present invention comprises a bag **11** having an aperture and an anatomically-shaped flange **12** surrounding the aperture for adhesive attachment to the perianal area of the wearer. The anatomically-shaped flange **12** is attached to the bag **11** according to means known to the man skilled in the art.

The anatomically-shaped flange **12** comprises a body-compatible adhesive that is covered with a release means (not shown) in order to protect the adhesive layer. The adhesive can cover the entire surface of the flange **12** or can partially cover the entire surface. As is evident from Figure 1, the adhesive is not applied to the whole surface area of the flange **12**. Lobes **13** on either side of the flange **12** remain free of adhesive to facilitate more easily the removal of the release means. Before application of the faecal management device **10** to the perianal area of the wearer, the release means is removed.

From Figure 1, it is evident that the anatomically-shaped flange **12** comprises an outer periphery **14**, an inner periphery **15** adjacent to the aperture, a longitudinal centreline **L** and a transverse centreline **T** orthogonal thereto. The transverse centreline **T** segments the anatomically shaped flange **12** into a front portion **16** and a rear portion **17**. The anatomically shaped flange **12** comprises a projection **18** in the front portion **16**. The presence of such a projection **18** is particularly suited to the contours of a female wearer since the projection **18** fits snugly between the vulva and the anus, i.e., the perineum. Furthermore, the flange **12** is optimally shaped, in particular, it is substantially heart-shaped in order to conform to the contours of the wearer when in use.

The projection **18** is disposed between the outer periphery **14** and the inner periphery **15** of the anatomically-shaped flange **12** in a direction parallel to the longitudinal direction **L**. More preferably, the flange **12** extends from the outer periphery **14** to the inner periphery **15**. In yet even a more preferred embodiment, the projection **18** is disposed in a symmetrical manner. It is important that the projection **18** be upstanding and rise above the plane of the flange **12** to an effective height **H** sufficient to present an abrupt discontinuity to obstruct the movement of the faecal material into the urethra of the female wearer. As used herein, the term "effective height" refers to the distance in the Z-direction from the surface of the flange **12**. The projection **18** has an effective height ranging from 2 millimetres to 20 millimetres. Preferably, the projection **18** has an effective height ranging from 3 millimetres to 10 millimetres, more preferably an effective height of about 5 millimetres. Typically, the projection **18** is orthogonal to the plane of the flange **12**. It should be recognised that if the flange **12** has wrinkles, rugosities, undulations or other deviations from planarity, these should be taken into account at the position of the projection **18** when determining its effective height. The projection **18** may either be coated with adhesive or be free from adhesive. According to a preferred embodiment of the present invention, the projection **18** is covered with a body-compatible adhesive.

The projection **18** may be made by forming a pleat in the constituent material of the flange **12** or by attaching a different material or a material similar to the flange material to the surface of the flange **12**. In the embodiment of the present invention, the projection **18** is formed from the flange material itself. The material or materials used for the projection **18** and the flange **12** are typically compliant and resilient and can comprise foams such as polyurethane, cellulosic or polyether foams or open-celled absorbent polymeric foams known typically as HIPE-derived foams (HIPE being high internal phase water-in-oil emulsion), non-wovens, air-laid materials including natural and synthetic fibres, thermally bonded airlaid materials, felt fabrics, needlepunched fabrics, spunlaced fabrics, fluid jet entangled fabrics, wet-laid fabrics, dry-laid fabrics, melt-blown fabrics, staple fibre carding fabrics, spunbonded fabrics, stitch-bonded fabrics, apertured fabrics, combinations of the above or the like.

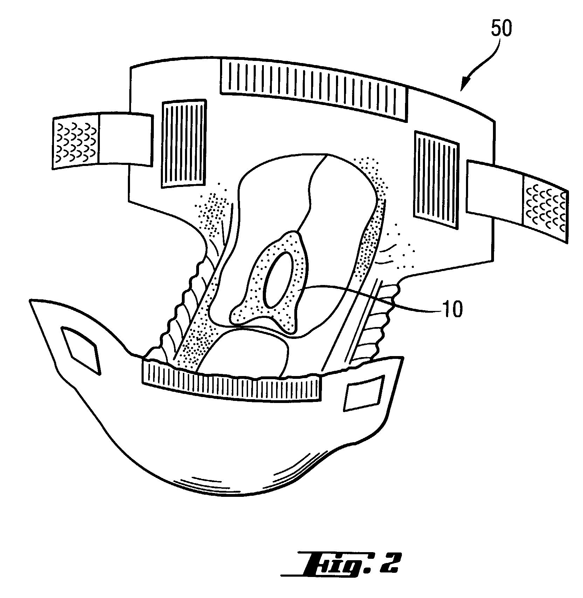

The faecal management device **10** of the present invention has been found to be particularly useful and beneficial when used in conjunction with a disposable diaper **50** - refer to Figure 2. The faecal management device **10** is first positioned in the perianal area of the female wearer with the projection **18** being fitted between the vulva and anus before the disposable diaper **50** is applied. In particular, the diaper **50** is positioned over the faecal management device **10** and fastened in a conventional manner around the body of the wearer. It has been found that, in addition, to providing excellent separation between urine and faecal material, the combined faecal management device **10** and diaper **50** system actually reduces skin irritation. In effect, the presence of the faecal management device **10** permits the formation of a separation layer between the skin of the wearer and the diaper **50**, i.e., a part of the absorbent core **58** of the diaper **10**. In particular, the projection **18** prevents the flow of faecal matter into the urethra of the female wearer, thus preventing a nasty infection. The diaper **50** can be of the conventional type (an embodiment of which is described below although not a limiting example by any means) or can be adapted to contain in an effective and comfortable manner the faecal management device **10** according to the teachings of the present invention.

As used herein, the term "disposable diapers" refers to articles which absorb and contain body exudates; and more specifically, refers to articles which are placed against or in proximity to the body of the wearer to absorb and contain the various exudates discharged from the body and which are intended to be discarded after a single use (i.e., they are not intended to be laundered or otherwise restored or reused) and, preferably, to be recycled, composted or otherwise disposed of in an environmentally compatible manner. As used herein, the term "diaper" refers to a garment generally worn by infants or incontinence sufferers that is drawn up between the legs and fastened about the waist of the wearer.

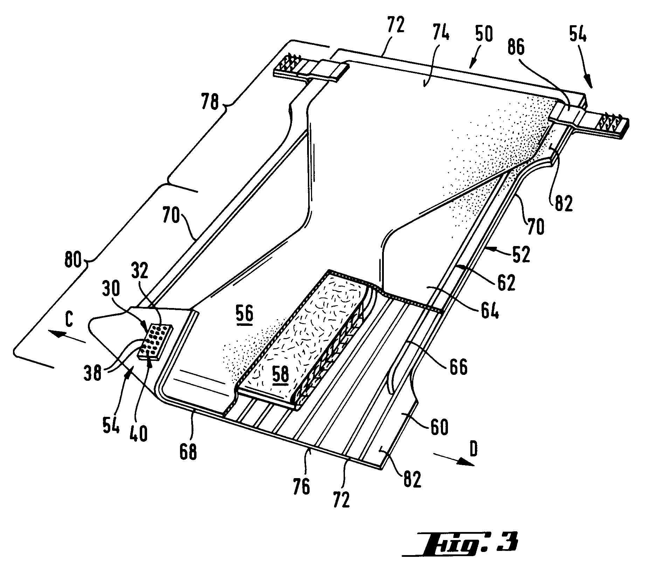

Figure 3 is a partially cut-away perspective view of a diaper **50** embodying the present invention prior to it being placed on the wearer over the faecal management device **10**. As is visible from Figure 3, a preferred diaper **50** comprises a body portion **52** and a refastenable mechanical fastening device **54**. A preferred body portion **52** comprises a liquid pervious topsheet **56**, an absorbent core **58**, a liquid impervious backsheet **60**, and elastically contractible leg cuffs **62**; each leg cuff **62** preferably comprising a side flap **64** and one or more elastic members **66**. For simplicity purposes, only one elastic member **66** is shown in the side flap **64**. While the topsheet **56**, the absorbent core **58**, the backsheet **60**, the side flaps **64**, and the elastic members **66** may be assembled in a variety of well-known configurations, a preferred disposable diaper configuration is shown and generally described in US 3,860,003. In this preferred diaper configuration, the backsheet **60** is joined to the topsheet **56**; the absorbent core **58** is positioned between the topsheet **56** and the backsheet **60**; the side flaps **64** extend outwardly from and along each side edge of the absorbent core **58**; and the elastic member **66** is operatively associated with each side flap **64**.

Figure 3 shows the body portion **52** in which the topsheet **56** and the backsheet **60** are coextensive and have length and width dimensions generally larger than those of the absorbent core **58**. The topsheet **56** is superposed on the backsheet **60** thereby forming the periphery **68** of the body portion **52**. The periphery **68** defines the outer perimeter or in other words the outer extent of the body portion **52**. The periphery comprises the longitudinal side edges **70** and lateral end edges **72**. In the longitudinal direction, the diaper **50** has a first end region **78** and a second end region **80**.

The body portion **52** has an inside surface **74** and an outside surface **76**. In general, the outside surface **76** of the diaper **50** extends from one edge **72** to the other end edge **72** of the diaper **50** and from one longitudinal side edge **70** to the other longitudinal side edge **70** of the diaper **50**. When a backsheet **60** is used, it typically forms the outside surface **76** of the body portion **52**. The inside surface **74** is that surface of the diaper **50** opposite the outside surface **76** and in the embodiment shown is typically formed by the topsheet **56**. In general, the inside surface **74** of the diaper **50** is that surface coextensive with the outside surface **76** and which is for the greater part in contact with the wearer when the diaper **50** is worn.

The diaper **50** has a first end region **78** and a second end region **80** extending from the lateral end edges **72** of the diaper periphery **68** towards the lateral centreline of the diaper **50**. Both the first end region **78** and the second end region **80** extend a distance of about one-half the length of the diaper **50** such that the end regions **78**, **80** comprise each half of the diaper **50**. Both the first end region **78** and the second end region **80** have panels **82**. The panels **82** are those portions of the first end region **78** and the second end region **80** which overlap when the diaper **50** is fastened about the waist of the wearer. The extent to which the end regions **78** overlap and thus the extent to which the panels **82** are formed, will depend on the overall dimensions and shape of the diaper **50** and the size of the wearer.

The absorbent core **58** of the body portion **52** may be any absorbent means which is generally compressible, conformable, non-irritating to the skin of the wearer, and capable of absorbing and retaining liquids such as urine and other certain bodily discharges. The absorbent core **58** may be manufactured in a variety of sizes and shapes (for example, rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core **58** may also be varied (for example, the absorbent core **58** may have varying caliper zones, hydrophilic gradients, superabsorbent gradients, or lower average density and lower average basis weight acquisition zones; or may comprise one or more layers or structures). The total absorbent capacity of the absorbent core **58** should, however, be compatible with the design loading and the intended use of the pull-on diaper. Further, the size and absorbent capacity of the absorbent core **58** may be varied to accommodate wearers ranging from infants to adults.

The backsheet **60** is impervious to liquids (for example, urine) and is preferably manufactured from a thin plastic film, preferably a thermoplastic film, although other flexible liquid impervious materials may also be used. As used herein, the term "flexible" refers to materials which are compliant and which will readily conform to the general shape and contours of the human body. The backsheet **60** prevents the exudates absorbed and contained in the absorbent core **58** from soiling articles which are in contact with the diaper **50** such as undergarments and bedding. The backsheet **60** may thus comprise polymeric films such as thermoplastic films of polyethylene or polypropylene, or composite materials such as film-coated non-woven material. For economic, aesthetic and ecological reasons, the backsheet **60** has an average nominal caliper, i.e., calculated caliper, of less than about 0.051 millimetres, more preferably a calculated caliper of from 0.020 millimetres to 0.036 millimetres. Preferably, the backsheet **60** is a flexible polyethylene film. As used herein, the term "polyethylene" film refers to films which are essentially made of polyethylene, however, it is understood that polyethylene film will contain a variety of additives to provide characteristics like opacity, strength requirements, colour, or any other desired characteristic that can be achieved through adding minor amounts of other substances than polyethylene into the films. The total amount of additives should be less than 45 percent, preferably less than 15 percent, by weight of film materials. Particularly, for opacity of the film, titanium dioxide is commonly used in a range of 2 to 6 percent, preferably 3.5 to 4.8 percent, by weight of the film. Exemplary films are manufactured by Tredegar Industries, Inc. of Terre Haute, Ind., USA or BP-Chemical PlasTec Rotbuchenstrasse 1, D-8000 München, Germany.

The backsheet **60** is preferably textured to provide a more clothlike appearance. Further, the backsheet **60** may also permit vapours to escape from the absorbent core **58** while still preventing exudates from passing through the backsheet **60** by, for example, being supplied with microapertures. The size of the backsheet **60** is dictated by the size of the absorbent core **58** and the exact diaper design selected.

The topsheet **56** of the body portion **52** of the present invention is compliant, soft feeling and non-irritating to the skin of the wearer. Further, the topsheet **56** is liquid pervious permitting liquids (for example, urine) to readily penetrate through its thickness. A suitable topsheet **56** may be manufactured from a wide range of materials, such as porous foams, reticulated foams, apertured films; or woven or non-woven webs of natural fibres (for example, wood or cotton fibres) or from a combination of natural and synthetic fibres. Preferably, it is made of a material that isolates the skin of the wearer from liquids retained in the absorbent core **58**.

There are a number of manufacturing techniques which may be used to manufacture the topsheet **56**. For example, the topsheet **56** may be a non-woven web of fibres. When the topsheet **56** comprises a non-woven web, the web may be spunbonded, carded, wet-laid, melt-blown, hydroentangled, hydroformed, combinations of the above, or the like. An exemplary topsheet **56** is carded and thermally bonded by means well-known to those skilled in the fabric art and comprise staple length polypropylene fibres having a denier of about 2.2 and has a basis weight from about 15 to about 30 grammes per square metre. As used herein, the term "staple length fibres" refer to those fibres having a length of at least 16 millimetres. A suitable topsheet **56** is manufactured by, for example, Veratec Inc., a division of International Paper Company, of Walpole, Mass., USA. A topsheet **56** particularly preferred for incontinence garments comprises a formed thermoplastic film.

### GLOSSARY

- 10: Faecal management device
- 11: Bag
- 12: Flange
- 13: Lobes
- 14: Outer periphery
- 15: Inner periphery
- 16: Front portion
- 17: Rear portion
- 18: Projection
- 50: Diaper
- 52: Body portion
- 54: Mechanical fastening device
- 56: Topsheet
- 58: Absorbent core
- 60: Backsheet
- 62: Leg cuffs
- 64: Side flaps
- 66: Elastic members
- 68: Periphery of body portion
- 70: Longitudinal side edges
- 72: Lateral end edges
- 74: Inside surface of body portion
- 76: Outside surface of body portion
- 78: First end region
- 80: Second end region
- 82: Panels
- L: Longitudinal centreline
- T: Transverse centreline

## Claims

1. Faecal management device (10) comprising a bag (11), said bag (11) having an aperture and an anatomically-shaped flange (12) surrounding said aperture for adhesive attachment to perianal area of wearer, said anatomically-shaped flange (12) being attached to said bag (11) wherein said anatomically-shaped flange comprises an outer periphery (14), an inner periphery (15) adjacent said aperture, a longitudinal centreline (L) and a transverse centreline (T) orthogonal thereto, said transverse centreline (T) segmenting said anatomically-shaped flange (12) into a front portion (16) and a rear portion (17),
characterised in that
said anatomically-shaped flange (12) comprises a projection (18) in said front portion (16).

2. Faecal management device (10) according to claim 1 wherein said projection (18) is disposed between said outer periphery (14) and said inner periphery (15) of said anatomically-shaped flange (12) in a direction parallel to said longitudinal direction (L).

3. Faecal management device (10) according to claim 2 wherein said projection (18) extends from said outer periphery (14) to said inner periphery (15).

4. Faecal management device (10) according to any of the preceding claims wherein said projection (18) is disposed in a symmetrical manner.

5. Faecal management device (10) according to any of the preceding claims wherein said projection (18) has an effective height ranging from 2 millimetres to 20 millimetres.

6. Faecal management device (10) according to claim 5 wherein said projection (18) has preferably an effective height ranging from 3 millimetres to 10 millimetres, more preferably about 5 millimetres.

7. Faecal management device (10) according to any of the preceding claims wherein said projection (18) is adapted to fit between vulva and anus of said female wearer.

8. Faecal management device (10) according to any of the preceding claims wherein said anatomically-shaped flange (12) is substantially heart-shaped.

9. Use of a faecal management device (10) according to any of the preceding claims in combination with a disposable diaper (50).

10. Use of a faecal management device (10) according to claim 9 whereby said faecal management device (10) is first positioned in the perianal area of a female wearer, said projection (18) being fitted between the vulva and anus and then said disposable diaper (50) is positioned over said faecal management device (10) and fastened in a conventional manner around body of said female wearer.

11. Use of a faecal management device (10) according to claims 9 and 10 wherein said faecal management device (10) provides a separation layer between skin of said female wearer and said disposable diaper (50).

12. Use of a faecal management device (10) according to claim 10 wherein said projection (18) prevents flow of faecal matter into urethra of said female wearer.
